# EUROPEAN PATENT SPECIFICATION

(11) **EP 2 590 611 B1**
(45) Date of publication and mention of the grant of the patent: **19.04.2017**
(21) Application number: 11803889.2
(22) Date of filing: 21.06.2011
(51) Int. Cl.: A61G 11/00, A61G 7/057, A61F 5/37

(54) **SECURING DEVICE FOR TRANSPORT OF NEONATAL PATIENTS IN A TEMPERATURE-CONTROLLED INCUBATOR**
VORRICHTUNG ZUR SICHERUNG DES TRANSPORTS NEUGEBORENER PATIENTEN IN EINEM BRUTKASTEN MIT TEMPERATURKONTROLLE
DISPOSITIF DE FIXATION POUR LE TRANSPORT DE PATIENTS NOUVEAU-NÉS DANS COUVEUSE À RÉGULATION DE TEMPÉRATURE

(30) Priority: 05.07.2010 SE 1050740
(43) Date of publication of application: 15.05.2013
(73) Proprietor: PA Consulting AB, 341 60 Ljungby (SE)
(72) Inventor: ANDERSSON, Pär, S-341 39 Ljungby (SE)
(74) Representative: Awapatent AB
(86) International application number: PCT/SE2011/050810
(87) International publication number: WO 2012/005658

(56) References cited:
- EP-A1- 0 088 704
- WO-A1-2009/006925
- WO-A2-2006/134363
- US-A- 4 509 505
- US-A- 4 885 811
- US-A- 5 626 150
- US-A- 5 906 205
- US-A1- 2003 139 694
- US-A1- 2004 244 114

## Description

### Field of the invention

The present invention relates to a securing device for transport of a neonatal patient in a temperature-controlled incubator.

### Prior art

In recent years, medical developments have enabled more and more prematurely born babies to be saved. They must then often spend their first weeks of life in an incubator. The medical expertise which is required to deal with the complications which can arise after the birth is often centralized within specialist units in larger hospitals, so that prematurely born incubator babies sometimes need to be transported. The transport is carried out in a tailor-made transport incubator, which can be provided with systems for, for example, maintaining a desired air temperature and air humidity, life-support systems, etc. A shorter or less urgent transport can be realized as a ground transport, for example in an ambulance; over longer distances and in more urgent cases, it can be carried out by air, for example in a helicopter.

Known transport incubators are marketed, *inter alia*, by Mansell Neonatal Transport Equipment and Atom Medical International.

Unfortunately, incubator babies within the size range 500-2500g have difficulty in surviving a crash or shock and can be seriously injured even under heavy braking. This is due, *inter alia*, to the fact that neither the skeleton nor the skin of an incubator baby is fully developed. A further aggravating factor is that the skin of the incubator baby cannot tolerate load, so that it can injure the baby to clamp it in place with a safety belt or the like.

WO 2006/134363 describes a securing device for transport of neonatal patients. The securing device comprises a plurality of velcroed securing straps, which are intended to hold an incubator baby against a base plate. A plurality of supporting elements see to it that the pressure from the securing straps is alleviated, so that the delicate skin of the baby is not subjected to excessively high pressure from the straps.

US 4 885 811A discloses a restraint comprising a multiplicity of flexible bubbles which are encased in an air-tight envelope of film material. When air inside the envelope is withdrawn, the restraint molds to the shape of the object it surrounds, The restraint may be designed as an infant carrier,

There is a need, however, for a more practical securing device which reduces the risk of injury to the baby during transport.

### Summary of the invention

One object of the present invention is to provide safer transport facilities for neonatal patients. This object is achieved by means of a securing device for transport of a neonatal patient in a temperature-controlled incubator, which securing device comprises a vacuum mattress comprising a gas-tight mattress case enclosing a granular filler material, which vacuum mattress is arranged to be brought from a flexible state into a rigid state by evacuation of gas from the same, the vacuum mattress comprising a first side portion arranged to be folded up around a first side of the patient, a second side portion arranged to be folded up around a second side of the patient, and a bottom portion arranged to be fastened in an incubator. Such a securing device can protect the patient against shocks and, by being able to be fastened in an incubator, can also prevent the patient from being thrown around inside the incubator under fierce acceleration or heavy braking.

According to one embodiment, the bottom portion is provided with a rigid plate for securement of the bottom portion in an incubator. The plate distributes the forces to which the vacuum mattress is subjected over a large part of the bottom portion and thereby reduces the risk of the patient being subjected to harmful point loads. Moreover, the plate helps the vacuum mattress to maintain its shape under heavy braking. According to one embodiment, the said rigid plate is provided with a fastening device for fixing the securing device to an insert base for an incubator. As a result, an incubator can be quickly and easily furnished with a securing device upon requirement.

According to one embodiment, the mattress case comprises a PVC-based plastics sheeting which is non-toxic at 38°C.

According to one embodiment, the mattress case is enclosed by a purpose-sewn mattress cover made of gas-permeable and moisture-permeable fabric. This produces a soft surface which has low friction in contact with skin and which allows the skin to be ventilated. Moreover, such a cover can be removed from the vacuum mattress and be washed with other fabrics.

According to one embodiment, the vacuum mattress comprises a leg portion arranged to be folded up around the feet and legs of the patient transversely to the longitudinal direction of the patient. A more stable transport securement of the patient can thereby be achieved.

According to one embodiment, each of the said first and second side portions is provided with a respective row of tensioning strap through holes for fastening of a safety harness. A more stable transport securement of the patient can thereby be achieved. Preferably, a shortest distance between the said respective rows of tensioning strap through holes is between 20 cm and 35 cm when the vacuum mattress is in the flat state. This distance gives a suitable size of vertical component of the contact force of the safety harness against the patient when the securing device is in the evacuated position, so that a good balance between securing capacity and pressure relief is achieved.

According to one embodiment, the securing device comprises a safety harness comprising an integrally joined transparent cloth, which, during use, is arranged to cover at least 50% of the trunk and head of the patient. Such a harness gives a more stable transport securement of the patient, at the same time as it allows the condition of the patient to be monitored since it is possible to see through the cloth. Preferably, the whole of the head and at least 50% of the trunk are covered.

### Brief description of the drawings

The invention will now be described in detail with reference to the appended drawings, in which:
Fig. 1a is a schematic perspective view, with broken-off parts, of a securing device for transport of neonatal patients;
Fig. 1b is a schematic perspective view of the securing device in Fig. 1a, wherein the securing device is provided with a safety harness;
Fig. 1c is a schematic side view of the securing device in Fig. 1b in a folded-up position; and
Fig. 2 is a schematic perspective view of a transport incubator provided with a securing device.

### Description of preferred embodiments

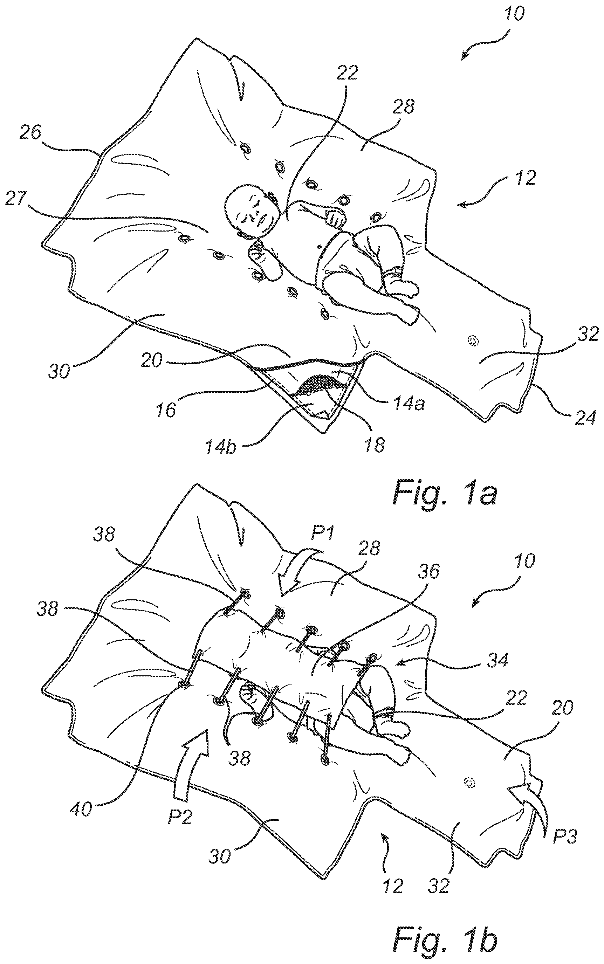

Fig. 1a illustrates a securing device 10 comprising a vacuum mattress 12. The vacuum mattress 12, which in Fig. 1a is in a flexible state, assumes a substantially flat shape when it is spread out on a plane surface and it is in the flat shape that it is shown in Fig. 1a. The vacuum mattress 12, which in Fig. 1a is shown partially in cross section, comprises an upper, flexible plastics sheeting piece 14a, which along its periphery 16 is welded together with a lower, likewise flexible plastics sheeting piece 14b, so as thereby to form a gas-tight mattress case. A suitable plastics material for the mattress case is, for example, non-rigid polyvinyl chloride (PVC) plastic. The mattress case is at least partially filled with granular filler material 18, such as, for example, styrofoam granulate. The vacuum mattress 12 is in its flat state preferably from 5 to 25 mm thick. A mattress cover 20 is purpose-sewn to and encloses the vacuum mattress 12. The mattress cover 20 is preferably pressure-relieving, so that any folds in the upper plastics sheeting piece 14a do not risk injuring a patient, and also air and moisture-permeable, so that it allows moisture to be transported away from the patient. The mattress cover 20 gives a soft contact surface and friction against the skin which is low in relation to the plastics sheeting 14a-b of the vacuum mattress 12. According to one embodiment, the mattress cover 20 comprises two layers: a first, pressure-relieving cover layer, close to the vacuum mattress, and a second, outer cover layer, close to the skin. The pressure-relieving cover layer can be given a good pressure-relieving effect by a combination of polyester and lycocell, for example in the proportions of approximately 57% polyester and approximately 43% lycocell. For the skin-close cover layer, a cotton and polyethylene knitted fabric, which can be knitted in the proportions, for example, of approximately 58% polyester and approximately 42% cotton, is suitable, the skin-close cover layer being formed with a polyester side facing the patient, so as to give a dry contact surface which does not cool the patient and a cotton side facing the pressure-relieving cover layer for the evacuation of moisture.

The securing device 12 is arranged to be fitted around a neonatal patient 22 in the manner illustrated in Figs. 1a-1c. By the term neonatal patient is meant a prematurely born infant weighing between 500 and 2500 grams.

In Fig. 1a is illustrated how the patient 22 has been placed centrally on the vacuum mattress 12. In Fig. 1a, the patient 22 has been placed so that she is lying on her back, but can equally well be placed, for example, lying on her side, on her stomach, or in the foetal position. An approximate longitudinal direction for the vacuum mattress 12 is defined by the longitudinal direction of the patient 22; the vacuum mattress 12 thus has a longitudinal direction running from a foot end 24 to a head end 26 of the vacuum mattress 12. The vacuum mattress 12 has a bottom portion 27, on which the patient 22 rests. The vacuum mattress 12 is further provided with a first side portion 28, a second side portion 30 and a leg portion 32. Both the side portions 28, 30 and the leg portion 32 are connected to the bottom portion 27. Preferably, the vacuum mattress 12 has in the flat state a length, in its longitudinal direction, ranging from 60 to 90 cm, and more preferably from 70 to 80 cm. In addition, the vacuum mattress 12 has in the flat state preferably a width, in a transverse direction running at right angles to the longitudinal direction in the plane of the vacuum mattress, ranging from 35 to 55 cm, and more preferably from 40 to 50 cm.

In Fig. 1b is illustrated how a safety harness 34 has been placed over the patient 22. The safety harness 34 comprises a cloth 36, which preferably is transparent, i.e. possible to see through, so that a carer can observe the patient 22 through the cloth 36 during transport. In the shown example, the cloth 36 of the safety harness 34 extends over approximately half the length of the patient and also covers the head of the patient 22. On small children, the mass of the head constitutes a substantial part of the total body mass; it is therefore desirable that a good securement of the head is ensured. However, it is then also desirable for the cloth 36 to afford good air permeability, especially if air is supplied by CPAP (Continuous Positive Airway Pressure).

The cloth 36 is anchored in the vacuum mattress 12 by means of a plurality of tensioning straps 38, which are fastened in the cloth 36 and run through respective through holes 40 in the vacuum mattress 12 and the mattress cover 20. The holes 40 are arranged in two rows running in the longitudinal direction of the vacuum mattress 12, with a row being situated in each of the side portions 28, 30. Preferably, the two rows are placed at a mutual distance apart of between 20 cm and 35 cm, and most preferably approximately 27 cm.

In a first step, the patient 22 is secured by the said first side portion 28 being folded up around a first side of the patient 22, as shown with an arrow P1; the said second side portion 30 is folded up around a second side of the patient 22, which in Fig. 1b is illustrated with an arrow P2; and the said leg portion 32 is folded up around the legs of the patient 22 in the direction illustrated with an arrow P3, i.e. the fold or the curve which is thereby produced in the leg portion extends transversely to the longitudinal direction of the patient.

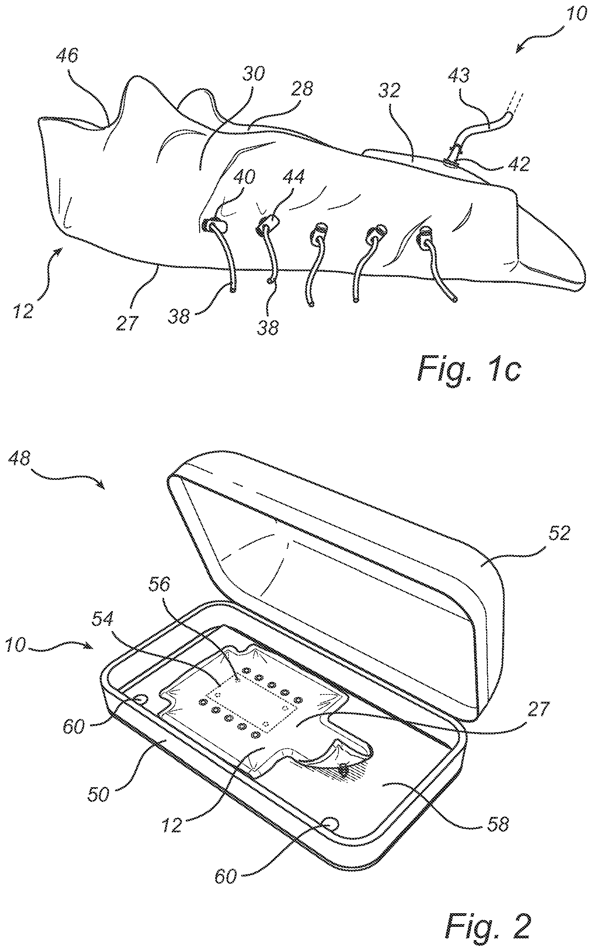

The securing device 10 has now assumed the folded-up shape shown in Fig. 1c. The first securing step is completed by the evacuation of gas from the vacuum mattress 12, for example by gas being sucked out of an evacuation valve 42 disposed in the leg portion 32 of the vacuum mattress 12. The evacuation of the vacuum mattress 12 sucks together the gas-tight mattress case 14a-b of the vacuum mattress 12, which compresses the granulate in the interior of the vacuum mattress 12 so that the vacuum mattress 12 rigidifies in the shape in which it was placed prior to evacuation, i.e. in the shape which is illustrated in Fig. 1c. Preferably, the vacuum mattress 12 is evacuated via the evacuation valve 42 by means of a vacuum pump (not shown), which, when an evacuation exists, is connected to the vacuum valve 42 with a tube 43. Preferably, both the side portions 28, 30 and the bottom portion 27 and leg portion 32 are arranged to be evacuated via the same evacuation valve 42, even though the vacuum mattress 12 can alternatively be provided with a plurality of evacuation valves for the different pieces 27, 28, 30, 32.

After the first securing step, the vacuum mattress 12 has formed a rigid, protective shell, which encloses the patient 22 (Fig. 1b) and absorbs shocks. Through the choice of granulate 18, the resilience of the vacuum mattress 12 can to a certain degree be adjusted, so that its shock-absorbing capacity can thereby be specially adapted to different transport conditions. Styroform granulate has been shown to give suitable resilience for good shock absorbency under those conditions which prevail during ambulance transports of neonatal patients 22.

The capacity of the vacuum mattress 12 to be shaped to the body of the patient 22 also means that the securing device 10 is well constituted to absorb shearing forces upon the patient 22 during braking. This is desirable, since the non-fully developed skin of the patient 22 is especially sensitive to shearing forces.

The side pieces are provided with recesses 46, which facilitate shaping of the vacuum mattress 12 to the body and head of the patient 22. The recesses 46 also facilitate the running of tubes to and from the patient 22, for example CPAP, when the vacuum mattress 12 is evacuated. Similar recesses are also arranged in the leg portion 32.

After the side portions 28, 30 of the vacuum mattress 12 have been folded up, the rows of through holes 40 of the vacuum mattress 12 are located on both sides of the patient 22.

In a second step, the patient 22 is secured by careful tightening of each of the tensioning straps 38 by means of a respective cord lock 44, so that the safety harness 34 is brought into contact with the patient 22. The securement of the patient in the vertical direction is thereby reinforced, so that she is not at risk of being thrown out of the shaped, rigidified vacuum mattress 12. At the same time, the safety harness 34 affords the patient 22 a certain mobility.

In order to produce better wear resistance of the tensioning belt through holes 40 in the mattress cover, these can be provided with eyelets or can have edges sewn with buttonhole seams. The corresponding tensioning strap through holes 40 of the vacuum mattress 12 can also be provided with eyelets; over and above their wear-resistance-enhancing effect, they can also in the vacuum mattress 12 help to distribute the tensile stress of the tensioning straps 38 within the vacuum mattress 12, which can lessen the mechanical load upon the patient during braking. The eyelets of the vacuum mattress 12 are preferably made of plastic, which reduces the risk of wear and tear which might jeopardize the gas-tightness of the vacuum mattress 12.

Fig. 2 shows a transport incubator 48 provided with a securing device 10 for transport securement of a neonatal patient 22 (Fig. 1a). The securing device 10 is provided with a vacuum mattress 12, which can have, for example, the configuration described in detail above. The vacuum mattress 12 is illustrated in Fig. 2 in its flexible, non-evacuated state.

The incubator 48, which can be, for example, any transport incubator found on the market, comprises an incubator sub-frame 50 and a transparent incubator lid 52. The transport incubator 48 is connected in a non-illustrated manner to apparatus for maintaining a well-defined incubator environment, such as devices for regulating temperature, air humidity and oxygen content. The interior of the incubator 48 is preferably arranged to maintain a temperature close to the body temperature of the patient 22, so that the patient 22 shall not suffer either hypothermia or hyperthermia during lengthy transports. By the term temperature-controlled incubator is meant an incubator in which a temperature within the range 32-39°C is actively maintained by heating or cooling. For the majority of transports, depending on the medical condition of the patient, a temperature within the range of approximately 34-38°C is especially preferred, with a temperature of approximately 38°C generally being most preferred.

Many transport incubators 48 are also provided with medical apparatus, such as equipment for CPAP, infusions, ventilator, monitoring and measuring of SpO2 (oxygen saturation of haemoglobin).

The bottom portion 27 of the vacuum mattress 12 is provided with a rigid, rectangular plate 54 (in dashed representation in Fig. 2) made of rigid plastic, which plate 54 is welded in the vacuum mattress 12 between the upper and lower plastics sheeting pieces 14a, 14b thereof (Fig. 1a). The plate 54 is provided with fastening devices in the form of insertion screw threads 56 (dashed representation), to which access is afforded from the underside of the vacuum mattress 12. The vacuum mattress 12 can hence be screwed from below into an insert base 58 in the transport incubator 48.

A transport incubator 48 can represent a substantial investment cost and it is therefore desirable that the same transport incubator 48 can be furnished for different applications according to requirement. The insert base 58 is therefore arranged to be detachably mounted in the sub-frame 50 of the incubator 48, so that a patient 22 (Fig. 1a) can, if necessary, be secured for transport. When, for medical reasons, for example, a transport needs to be carried out without a securing device 10, then the securing device 10 can be easily removed from the transport incubator 48 by the removal of the insert base 58 or the exchange thereof for an insert base adapted to another purpose. In order to facilitate removal, the insert base 58 is provided with oblique through holes 60, through which care staff are able with their fingers to grasp the bottom side of the insert base 58.

That the securing device 10 is fixed to the sub-frame 50 of the transport incubator 48 reduces the risk of injury to the patient 22 should the incubator 48 be subjected to external influence, such as air pockets or braking actions. Furthermore, in the transport of an unsecured patient, tubes, intubations and other equipment are at risk during braking, for example, of rupturing internal blood vessels or of otherwise injuring the patient. By virtue of the fact that the securing device 10 is fixed in the incubator 48, this risk is also reduced, since the movement of the patient under heavy braking is minimized. By fixing any intubations in the recesses 46, the risks of such injuries can be further reduced.

The insert base 58 can be given a different configuration in order to fit in the transport incubators of different manufacturers.

Preferably, the mattress case 14a-b of the vacuum mattress 12 (Fig. 1) is formed of a material which does not give off toxic gases within the temperature range 32-39°C. The securing device 10 can hence be located in a temperature-controlled transport incubator 48 without releasing gases which are toxic to the patient 22. Since the vacuum mattress 12 is allowed to assume normal temperature, nor is there a need for a separate warming mattress for the patient 22. As an example of suitable non-toxic materials for the vacuum mattress 12 can be cited non-rigid polyvinyl chloride (PVC) plastics. Sheeting product no. 2702, which is supplied by Gislaved Folie AB, with the address Box 518, SE-332 28 Gislaved, Sweden, is a specific example of a suitable non-toxic plastics sheeting for the sheeting pieces 14a-b. This sheeting is calendered, which gives it a smooth surface which allows low friction against the mattress cover 20 and thus also reduced shearing forces against the skin of the patient 22 during braking. Furthermore, it is free from flame retardant, which contributes to its non-toxic properties at temperatures of up to 60°C.

PVC sheeting can also be washed with medical spirit, which makes it possible to reuse the securing device 10 without increased risk of infection.

It will be appreciated that a number of variants of the above-described embodiments are possible within the scope of the appended patent claims. For example, above a securing device 10 mounted in a transport incubator 48 has been described, which securing device is provided with a safety harness 34 and a mattress cover 20. It will be appreciated that the securing device can be used with or without the safety harness, with or without the mattress cover, and can be used, moreover, for transport in other types of incubators than purpose-built transport incubators. In this respect, all sorts of incubators which are used for transport can be regarded as transport incubators.

Furthermore, the vacuum mattress 12 has been described as comprising a first side portion 28 and a second side portion 30. The vacuum mattress 12 can, of course, comprise further side portions, for example by each of the above-described side portions 28, 30 being divided into a plurality of smaller side portions.

This application can come to constitute a basis for spin-off patent applications; especially as it will be appreciated that the securing device 10 can also be used to transport infants who are not born prematurely, and can also be used in the transport of children with no incubator or care requirements, for example by the securing device 10 instead being clamped in a conventional child car seat or a so-called "baby protector", i.e. a child car seat for very small infants. In these cases, of course, no incubator is needed.

## Claims

1. Securing device (10) for transport of a neonatal patient in a temperature-controlled incubator, which securing device comprises
a vacuum mattress (12) comprising a gas-tight mattress case (14a-b) enclosing a granular filler material (16), which vacuum mattress (12) is arranged to be brought from a flexible state into a rigid state by evacuation of gas from the same, the vacuum mattress (12) comprising a first side portion (28) arranged to be folded up around a first side of the patient (22), a second side portion (30) arranged to be folded up around a second side of the patient (22), and a bottom portion (27) arranged to be fastened in an incubator (48), wherein
the bottom portion (27) is provided with a rigid plate (54) for securement of the bottom portion (27) in the incubator (48) **characterized by** said rigid plate (54) being provided with a fastening device (56) for fixing the securing device (10) to an insert base (58) for an incubator (48).

2. Securing device according to claim 1, wherein the mattress case (14a-b) comprises a PVC-based plastics sheeting which at 38°C is non-toxic.

3. Securing device according to any one of the preceding claims, wherein the mattress case (14a-b) is enclosed by a purpose-sewn mattress cover (20) made of gas-permeable and moisture-permeable fabric.

4. Securing device according to any one of the preceding claims, wherein the vacuum mattress (12) comprises a leg portion (32) arranged to be folded up around the feet and legs of the patient (22) transversely to the longitudinal direction of the patient.

5. Securing device according to any one of the preceding claims, wherein each of the said first and second side portions (28, 32) is provided with a respective row of tensioning strap through holes (40) for fastening of a safety harness (34).

6. Securing device according to claim 5, wherein a shortest distance between the said respective rows of tensioning strap through holes (40) is between 20 cm and 35 cm when the vacuum mattress (12) is in the flat state.

7. Securing device according to any one of the preceding claims, further comprising a safety harness (34) comprising an integrally joined transparent cloth (36), which, during use, is arranged to cover at least 50% of the trunk and head of the patient (22).

## Patentansprüche

1. Vorrichtung zur Sicherung (10) des Transports eines neugeborenen Patienten in einem temperaturgesteuerten Brutkasten, wobei diese Sicherungsvorrichtung umfasst:
eine Vakuummatratze (12), die einen gasdichten Matratzenbezug (14a-b) umfasst, der einen körnigen Füllstoff (16) umschließt, wobei die Vakuummatratze (12) dafür ausgelegt ist, durch Evakuierung von Gas aus derselben von einem flexiblen Zustand in einen steifen Zustand gebracht zu werden, wobei die Vakuummatratze (12) einen ersten Seitenabschnitt (28), der dafür ausgelegt ist, nach oben um eine erste Seite des Patienten (22) herum geklappt zu werden, einen zweiten Seitenabschnitt (30), der dafür ausgelegt ist, nach oben um eine zweite Seite des Patienten (22) herum geklappt zu werden, und einen unteren Abschnitt (27), der dafür ausgelegt ist, in einem Brutkasten (48) befestigt zu werden, umfasst, wobei
der untere Abschnitt (27) mit einer starren Platte (54) zur Sicherung des unteren Abschnitts (27) in dem Brutkasten (48) versehen ist,
**dadurch gekennzeichnet, dass** die starre Platte (54) mit einer Befestigungsvorrichtung (56) zum Befestigen der Sicherungsvorrichtung (10) an einem Einsatzunterteil (58) für einen Brutkasten (48) versehen ist.

2. Vorrichtung zur Sicherung nach Anspruch 1, wobei der Matratzenbezug (14a-b) eine Kunststofffolie auf PVC-Basis umfasst, welche bei 38°C ungiftig ist.

3. Vorrichtung zur Sicherung nach einem der vorhergehenden Ansprüche, wobei der Matratzenbezug (14a-b) von einer speziell dafür genähten Matratzenhülle (20) umschlossen ist, die aus gasdurchlässigem und feuchtigkeitsdurchlässigem Stoff hergestellt ist.

4. Vorrichtung zur Sicherung nach einem der vorhergehenden Ansprüche, wobei die Vakuummatratze (12) eine Beinabschnitt (32) umfasst, der dafür ausgelegt ist, quer zur Längsrichtung des Patienten nach oben um die Füße und Beine des Patienten (22) geklappt zu werden.

5. Vorrichtung zur Sicherung nach einem der vorhergehenden Ansprüche, wobei der erste und der zweite Seitenabschnitt (28. 32) jeweils mit einer jeweiligen Reihe von Spannband-Durchgangslöchern (40) zum Befestigen eines Sicherheitsgeschirrs (34) versehen sind.

6. Vorrichtung zur Sicherung nach Anspruch 5, wobei ein kürzester Abstand zwischen den jeweiligen Reihen von Spannband-Durchgangslöchern (40) zwischen 20 cm und 35 cm beträgt, wenn sich die Vakuummatratze (12) im flachen Zustand befindet.

7. Vorrichtung zur Sicherung nach einem der vorhergehenden Ansprüche, welche ferner ein Sicherheitsgeschirr (34) umfasst, welches ein angeformtes durchsichtiges Tuch (36) umfasst, welches während des Gebrauchs dafür ausgelegt ist, wenigstens 50 % des Rumpfes und Kopfes des Patienten (22) zu bedecken.

## Revendications

1. Dispositif de fixation (10) pour le transport d'un patient nouveau-né dans une couveuse à régulation de température, ledit dispositif de fixation comprend
un matelas coquille à dépression (12) comprenant une enveloppe de matelas étanche aux gaz (14a-b) renfermant un matériau de remplissage granulaire (16), ledit matelas coquille à dépression (12) est agencé pour passer d'un état flexible à un état rigide par évacuation du gaz de celui-ci, le matelas coquille à dépression (12) comprenant une première partie latérale (28) agencée pour être repliée autour d'un premier côté du patient (22), une seconde partie latérale (30) agencée pour être repliée autour d'un second côté du patient (22) et une partie inférieure (27) agencée pour être fixée dans une couveuse (48), dans lequel
la partie inférieure (27) est dotée d'une plaque rigide (54) pour la fixation de la partie inférieure (27) dans la couveuse (48) **caractérisé par le fait que** ladite plaque rigide (54) est dotée d'un dispositif de fixation (56) pour fixer le dispositif de fixation (10) à un insert de base (58) pour une couveuse (48).

2. Dispositif de fixation selon la revendication 1, dans lequel l'enveloppe de matelas (14a-b) comprend une gaine en plastique à base de PVC qui n'est pas toxique à 38°C.

3. Dispositif de fixation selon l'une quelconque des revendications précédentes, dans lequel l'enveloppe de matelas (14a-b) est protégée par une housse de matelas cousue spécialement (20) en un tissu perméable aux gaz et perméable à l'humidité.

4. Dispositif de fixation selon l'une quelconque des revendications précédentes, dans lequel le matelas coquille à dépression (12) comprend une partie jambes (32) agencée pour être repliée autour des pieds et des jambes du patient (22) transversalement à la direction longitudinale du patient.

5. Dispositif de fixation selon l'une quelconque des revendications précédentes, dans lequel chacune desdites première et seconde parties latérales (28, 32) est dotée d'une rangée respective de trous de passage de sangle de tension (40) pour fixer un harnais de sécurité (34).

6. Dispositif de fixation selon la revendication 5, dans lequel une distance plus courte entre lesdites rangées respectives de trous de passage de sangle de tension (40) est de 20 cm à 35 cm lorsque le matelas coquille à dépression (12) est dans l'état plat.

7. Dispositif de fixation selon l'une quelconque des revendications précédentes, comprenant en outre un harnais de sécurité (34) comprenant une toile transparente liée d'un seul tenant (36), qui, durant l'utilisation, est agencée pour couvrir au moins 50 % du tronc et de la tête du patient (22).
